# FASCICULE DE BREVET EUROPEEN

(11) **EP 1 446 058 B1**
(45) Date de publication et mention de la délivrance du brevet: **17.06.2009**
(21) Numéro de dépôt: 02793236.7
(22) Date de dépôt: 14.11.2002
(51) Int. Cl.: A61B 17/12

(54) **DISPOSITIF D'OCCLUSION D'UN CONDUIT CORPOREL, NOTAMMENT D'UNE VEINE VARIQUEUSE**
VERSCHLUSSVORRICHTUNG FÜR EINE KÖRPERRÖHRE, INSBESONDERE VON KRAMPFADERN
DEVICE FOR OCCLUSION OF A CORPOREAL DUCT, IN PARTICULAR A VARICOSE VEIN

(30) Priorité: 19.11.2001 FR 0114803
(43) Date de publication de la demande: 18.08.2004
(73) Titulaire: SOFRADIM PRODUCTION, 01600 Trévoux (FR)
(72) Inventeur: DARNIS, Thierry, F-01480 Frans (FR); MENEGHIN, Alfredo, F-69480 Anse (FR); MILLERET, René, F-34000 Montpellier (FR); THERIN, Michel, F-69004 Lyon (FR)
(74) Mandataire: Brédeville, Odile Marie
(86) Numéro de dépôt international: PCT/FR2002/003903
(87) Numéro de publication internationale: WO 2003/043506

(56) Documents cités:
- EP-A- 0 800 791
- WO-A-94/09705
- WO-A-94/15534
- US-A- 5 192 301
- US-A- 5 310 407
- US-A- 5 690 666

## Description

La présente invention concerne un dispositif d'occlusion d'un conduit corporel, notamment d'une veine variqueuse. L'invention concerne également un dispositif de mise en place de ce dispositif d'occlusion dans ce conduit corporel.

Pour le traitement d'une veine variqueuse, la technique dite de "stripping" est habituellement pratiquée. Cette technique consiste à arracher la veine au moyen d'un câble ou d'une sonde. Elle a toutefois pour inconvénient d'être douloureuse et relativement exigeante quant à sa mise en oeuvre.

Des techniques de traitement endo-veineux ont également été envisagées.

La technique dite "CLOSURE" utilise une sonde émettant des ondes radio, ces ondes provoquant un échauffement de la paroi de la veine, et donc une lésion de celle-ci, de laquelle il résulte l'oblitération de la veine. Cette technique permet un bon contrôle du geste opératoire et de la température appliquée localement, et est utilisable pour des veines allant jusqu'à 12 millimètres de diamètre. Elle présente par contre le risque de brûlure de la peau si le patient est maigre, et nécessite une anesthésie générale ou locale avec une sédation forte compte tenu des douleurs qu'elle implique. De plus, cette technique n'est pas indiquée si la veine présente des zones fortement dilatées, n'est pas utilisable sur les veines collatérales et sur la veine saphène externe, ni sur les récidives, et a un coût de mise en oeuvre relativement élevé.

La technique dite "EVLT" utilise une fibre optique reliée à un laser. Cette technique est plus simple à mettre en oeuvre et moins coûteuse. Elle présente toutefois des risques de brûlure et implique des suites plus douloureuses que la technique précédente, compte tenu de la température de traitement, plus élevée. En outre, cette technique n'est pas utilisable pour certaines veines, telles que les veines anévrismales, la veine saphène externe ou les veines collatérales, et le laser a un coût élevé.

Une autre technique consiste à provoquer une sclérose de la veine par introduction d'un produit sous forme de mousse. Cette technique a un coût de mise en oeuvre réduit et permet de traiter les veines collatérales. Elle a toutefois pour inconvénient de ne pas permettre de contrôler la répartition du produit sclérosant, ce de quoi il résulte des risques plus importants de phlébite profonde et d'embolie. De plus, cette technique est moins efficace en termes d'obturation de la veine, étant donné qu'elle n'agit pas au niveau du collagène pariétal comme les techniques précédentes mais uniquement sur la zone endo-veineuse. En outre, la fiabilité de l'occlusion obtenue est incertaine dans le temps, des reperméations de la veine traitée étant relativement fréquentes à moyen terme.

WO 94/15534 décrit un dispositif d'occlusion d'un conduit corporel sous la forme d'un élément tubulaire comprenant des fibres, une portion de ces fibres étant radiopaque.

WO 94/09705 décrit un dispositif d'occlusion d'un conduit corporel sous la forme d'un élément tubulaire comprenant des fibres, ledit élément tubulaire enfermant une bobine hélicoïdale en matériau radiopaque.

La présente invention vise à remédier à l'ensemble des inconvénients des techniques existantes.

Son objectif principal est donc de fournir un dispositif d'occlusion implantable selon une procédure relativement simple, rapide et peu onéreuse à mettre en oeuvre, pouvant être mise en oeuvre sous anesthésie locale, et n'induisant que peu de douleurs post-opératoires et pas de complications au niveau des tissus, en particulier au niveau des nerfs péri-saphéniens.

Un autre objectif de l'invention est de fournir un dispositif d'occlusion présentant des possibilités d'utilisation élargies, c'est-à-dire pouvant notamment être utilisé pour l'occlusion de la veine saphène interne mais aussi de la veine saphène externe et des veines collatérales de gros calibre.

Ces objectifs sont atteints par un dispositif d'occlusion formé par plusieurs fils tricotés ensemble de manière à constituer un tricot allongé "cylindrique", c'est-à-dire un tricot dans lequel les fils formant les mailles se croisent sensiblement au niveau de la zone radialement interne de ce tricot.

Le dispositif selon l'invention a ainsi une structure à la fois maillée, faisant qu'il est compressible radialement et très souple longitudinalement, et "cylindrique", faisant que les fils sont présents au niveau de la zone radialement interne du dispositif. La compressibilité radiale et la souplesse du dispositif permettent l'introduction facile de ce dispositif dans le conduit à traiter, sous une simple anesthésie locale, et ne génèrent aucune rigidité sensible sous la peau ; la présence des fils au niveau de la zone radialement interne du dispositif, sur l'ensemble de la longueur du dispositif, permet d'assurer une occlusion fiable du conduit, sur une grande longueur de celui-ci. Dans le cas d'une veine variqueuse, le dispositif d'occlusion ne permet pas le passage direct du sang sur une longueur supérieure à une ou à quelques mailles ; il en résulte un ralentissement du flux sanguin conduisant à une thrombose de la veine, qui s'organise progressivement en fibrose.

Le dispositif d'occlusion selon l'invention n'implique ainsi aucun échauffement du conduit corporel traité et n'induit aucun risque de lésion des nerfs sensitifs qui accompagnent les veines. Il peut dès lors être implanté selon une procédure relativement simple, rapide et peu onéreuse à mettre en oeuvre, et peut être utilisé pour les indications les plus larges, en particulier pour traiter les veines Saphènes externes, les récidives et les veines collatérales.

De préférence, le dispositif est constitué de fils en matériau résorbable, notamment en acide polylactique, polyglycolique ou un copolymère de ces derniers.

Grâce à la structure maillée précitée, le dispositif d'occlusion a un rapport poids/volume très faible (de l'ordre de 0,02 à 0,08 grammes par cm³ selon les différentes tailles de dispositifs), qui lui permet d'occuper l'ensemble du volume du conduit avec une faible quantité de fil par unité de longueur. Cette faible quantité de fil rend possible une résorption complète du dispositif d'occlusion, sans réaction inflammatoire cliniquement significative. Dans le cas du traitement d'une veine variqueuse, une fois la fibrose de la veine réalisée, les macrophages résorbent les résidus hydrolysés du dispositif d'occlusion et les thrombis fibreux ; la veine se réduit finalement à un cordon fibreux qui sera éliminé par l'organisme, tout comme est éliminée l'acide lactique et/ou glycolique.

Avantageusement, le matériau résorbable constituant les fils du dispositif d'occlusion, le nombre de ces fils et le diamètre de ces fils sont déterminés de telle sorte que la durée nécessaire à la résorption du dispositif d'occlusion soit supérieure ou égale à la durée de résorption naturelle du conduit corporel traité une fois l'occlusion réalisée.

Le dispositif d'occlusion peut être réalisé en mailles jetées ou cueillies, et est de préférence réalisé en mailles jetées.

Avantageusement, les fils formant le dispositif d'occlusion sont enduits d'une ou plusieurs substances de nature à favoriser l'occlusion et/ou la dégénérescence du conduit corporel traité, ou reçoivent un traitement de surface augmentant leur thrombogénéité. Il peut notamment s'agir d'un produit propre à irriter la paroi du conduit pour induire un spasme de celui-ci autour du dispositif. Dans le cas du traitement d'une veine variqueuse, ce spasme favorise l'occlusion de la veine et l'apparition rapide de la-thrombose. Il peut s'agir également d'activateurs de la coagulation.

Le dispositif d'occlusion peut en outre comprendre au moins un fil longitudinal relié à ses extrémités, formant un moyen anti-allongement de ce dispositif lors de son introduction dans le tube longitudinal du dispositif de mise en place et lors de sa mise en place dans le conduit corporel.

Le dispositif de mise en place du dispositif d'occlusion précité comprend, selon l'invention, un tube longitudinal propre à contenir le dispositif d'occlusion au moment de l'implantation, et des moyens de préhension sont prévus pour permettre la préhension du dispositif d'occlusion au niveau d'une extrémité de ce tube, à savoir l'extrémité distale du tube par rapport à l'orifice par lequel ce tube a été introduit dans le conduit corporel à traiter. Le dispositif d'occlusion peut être préalablement chargé dans un tube longitudinal d'introduction ou bien être "chargé" extemporanément par le praticien juste avant la mise en place dans le conduit corporel du patient. Dans ce deuxième cas, le dispositif de mise en place comprend un système permettant la compression et l'introduction du dispositif d'occlusion dans le tube longitudinal.

Le tube contenant le dispositif d'occlusion est introduit dans le conduit corporel à traiter puis lesdits moyens de préhension sont utilisés pour saisir le dispositif d'occlusion sous la peau et maintenir ce dispositif d'occlusion pendant que le tube est retiré.

La mise en place du dispositif d'occlusion est ainsi particulièrement facile et rapide à réaliser. De plus, le tube a pour avantage de réaliser une relative agression de la paroi du conduit, qui s'ajoute à la relative agression de cette paroi provoquée ensuite par le dispositif d'occlusion lui-même. Dans le cas d'une veine variqueuse, cette agression contribue à l'apparition rapide de la thrombose.

Le tube est réalisé en une matière suffisamment rigide pour permettre l'engagement de ce tube dans la veine par coulissement mais suffisamment souple pour ne pas être blessant. Cette matière peut être par exemple du polyéthylène, du PTFE, du polyuréthanne et plus généralement tout polymère utilisé dans le domaine médical pour la confection de cathéters.

Avantageusement, le diamètre du tube est inférieur à celui du dispositif d'occlusion à l'état non comprimé de ce dernier.

Le dispositif d'occlusion permet ainsi de déposer dans le conduit corporel un dispositif d'occlusion ayant, à l'état non comprimé, un diamètre équivalent ou légèrement supérieur à celui de ce conduit corporel.

Il est ainsi obtenu une densité suffisante de fil dans le conduit corporel, qui assure l'occlusion du conduit dans les meilleures conditions, et un degré d'agression de la paroi du conduit qui contribue efficacement à cette occlusion.

Le diamètre du dispositif d'occlusion peut notamment aller de 4 à 9 mm selon les veines à traiter, et le diamètre externe du tube peut aller de 3 à 4,5 mm.

Les moyens de préhension peuvent être constitués à partir des fils formant le dispositif d'occlusion, notamment par nouage ou autre regroupement, en particulier fusion, de ces fils au niveau de l'extrémité distale du dispositif d'occlusion. Ces moyens peuvent également être constitués par un embout atraumatique relié au dispositif d'occlusion, pouvant être monté à l'extrémité distale du tube. Après retrait du tube, cet embout est extrait du corps du patient par une incision distale et une séparation d'avec le dispositif d'occlusion.

L'extrémité proximale du tube peut comporter un embout du type embase, équipé d'une valve hémostatique et d'une voie latérale terminée par un robinet à deux ou trois voies afin de permettre une injection de fluide à l'intérieur du tube, soit préalablement à la mise en place du dispositif d'occlusion, soit pendant cette mise en place. Cette extrémité proximale du tube peut en variante comporter un connecteur de type "Y" avec des extrémités au standard "Luer lock".

Un produit propre à provoquer une sclérose de la veine peut notamment être injecté de cette façon.

Pour sa bonne compréhension, l'invention est à nouveau décrite ci-dessous en référence au dessin schématique annexé représentant, à titre d'exemples non limitatifs, deux formes de réalisation possibles du dispositif d'occlusion et du dispositif de mise en place de ce dispositif d'occlusion concernés.
La figure 1 est une vue de côté d'une portion du dispositif d'occlusion selon une première forme de réalisation, ce dispositif, tel que représenté, étant grossi environ quatre fois ;
la figure 2 est une vue très schématique des aiguilles d'un métier à tricoter à partir duquel ce dispositif est obtenu, et du parcours des fils alimentant ces aiguilles ;
la figure 3 est une vue de la grille de liage correspondante ;
la figure 4 est une vue en coupe longitudinale dudit dispositif de mise en place, ledit dispositif d'occlusion étant engagé à l'intérieur de ce dispositif de mise en place ;
la figure 5 est une vue à échelle agrandie d'une incision pratiquée dans une jambe, permettant d'exposer la partie proximale de la veine variqueuse à traiter;
la figure 6 est une vue de la jambe après engagement dans la veine à traiter du dispositif d'occlusion et du dispositif de mise en place ;
la figure 7 est une vue similaire à la figure 6, après retrait du dispositif de mise en place ;
la figure 8 est une vue du dispositif d'occlusion et du dispositif de mise en place selon la deuxième forme de réalisation, en coupe longitudinale ; et
la figure 9 est une vue du dispositif d'occlusion et du dispositif de mise en place similaire à la figure 8, le dispositif de mise en place présentant à son extrémité proximale un embout de connexion différent de celui du dispositif montré sur la figure 8.

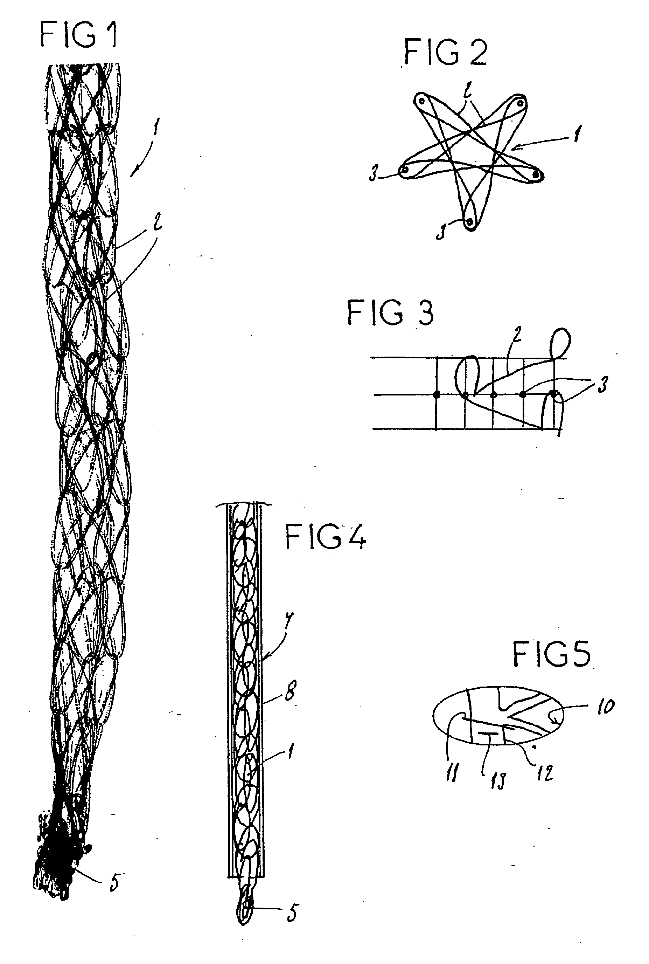

La figure 1 représente un dispositif d'occlusion 1 permettant le traitement d'une veine variqueuse.

On sait qu'une veine variqueuse est une veine qui n'est plus à même d'empêcher le reflux du sang en direction de l'extrémité du membre, c'est-à-dire en direction du pied dans le cas d'une jambe. Il en résulte un engorgement des tissus, et, lorsque la veine est très dilatée, le développement de thromboses.

La veine variqueuse doit alors être traitée de manière à empêcher la circulation du sang au travers d'elle ; les veines formant un réseau, le sang empruntera alors d'autres veines pour sa circulation.

Le dispositif 1 est formé par cinq monofils 2 en acide polylactique et/ou polyglycolique tricotés ensemble de manière à constituer un tricot allongé "cylindrique", c'est-à-dire un tricot dans lequel les fils 2 se croisent sensiblement au niveau de la zone radialement interne de ce tricot.

En référence aux figures 2 et 3, il apparaît que ce tricotage est réalisé sur un métier circulaire, qui est un métier chaîne. Ce tricotage est à mailles jetées. Le liage est obtenu en exécutant une grande amplitude avec les fils 2, permettant à ces derniers de sauter plusieurs aiguilles 3 ; un fil 2 alimente ainsi une aiguille 3 puis saute plusieurs aiguilles de manière à alimenter une seconde aiguille 3 distante angulairement de la première aiguille, de telle sorte que la portion de fil 2 située entre ces aiguilles s'étend au niveau de la zone radialement interne 4 de la surface circulaire délimitée par les aiguilles 3, qui est également la zone radialement interne du dispositif 1. Dans l'exemple représenté, le métier comprend cinq aiguilles 3 qui sont alimentées chacune par un fil 2. Par le mouvement du porte-fil, un fil 2 alimente une aiguille en maille ouverte puis saute deux aiguilles, de sorte qu'il alimente l'aiguille située en quatrième position par rapport à cette première aiguille, puis revient à la première aiguille, et le cycle recommence.

Comme cela apparaît sur la figure 1, le tricotin ainsi obtenu a une structure à la fois maillée, faisant qu'il est compressible radialement et très souple longitudinalement, et est "cylindrique", c'est-à-dire non tubulaire, faisant que les fils 2 sont présents au niveau de la zone radialement interne du dispositif 1, élément essentiel pour le caractère occlusif du dispositif 1.

A une extrémité du dispositif 1, les fils 2 sont réunis par exemple par thermofusion de manière à constituer une zone de préhension 5 de forme plus ou moins arrondie. Cette zone 5 est dimensionnée de manière à pouvoir être saisie au travers de la peau, ainsi que cela apparaîtra plus loin.

Le dispositif 1 est produit en une longueur suffisante pour permettent de traiter toute longueur de veine susceptible d'être affectée, par exemple 60 cm, et peut être coupé à la longueur désirée. Une thermofixation peut être réalisée pour limiter la déformabilité des mailles et éviter tout démaillage.

De plus, afin de faciliter ultérieurement l'introduction du dispositif d'occlusion 1 dans le dispositif 7 de mise en place, un ou plusieurs fils peuvent être "descendus" verticalement au centre du dispositif 1 pendant la phase de tricotage. Ces fils non maillés servent à limiter l'effet de la traction lors de l'introduction du dispositif d'occlusion 1 dans le dispositif de mise en place 7, et évitant ainsi une trop grande déformation des mailles. Ce ou ces fils longitudinaux évitent aussi l'allongement du dispositif 1 lors de son larguage dans la veine.

En référence à la figure 4, il apparaît que le dispositif 7 de mise en place du dispositif 1 comprend un tube 8, propre à contenir ce dispositif 1, avec toutefois la zone 5 dépassant d'une extrémité de ce tube 8. Ce tube 8 est en polyéthylène et a une épaisseur telle qu'il est suffisamment rigide pour permettre son engagement par coulissement dans la veine à traiter mais suffisamment souple pour ne pas être blessant et pour permettre une progression aisée dans les vaisseaux sinueux.

En pratique, ainsi que cela est illustré par les figures 5 à 7, une incision 10 est pratiquée dans la jambe de manière à exposer la jonction Saphèno-fémorale au Scarpa, ou à exposer la crosse Saphène externe derrière le genou ; une ligature 11 non résorbable est mise en place soit au niveau de la jonction Saphèno-fémorale si un reflux sanguin a été constaté à ce niveau, soit en dessous de cette jonction si l'on souhaite préserver le drainage des veine sous-cutanées abdominales et honteuses ; une incision 13 est ensuite réalisée dans la veine 12 à traiter, en dessous de la ligature 11, et le dispositif 7, dans lequel est introduit le dispositif 1 de la manière précitée, est engagé dans la veine 12 sur la longueur nécessaire ; une fois cet engagement réalisé, la zone 5 est saisie à travers la peau puis le tube 8 est retiré de manière à libérer le dispositif 1 dans la veine 12 ; l'incision 13 puis l'incision 10 sont refermées.

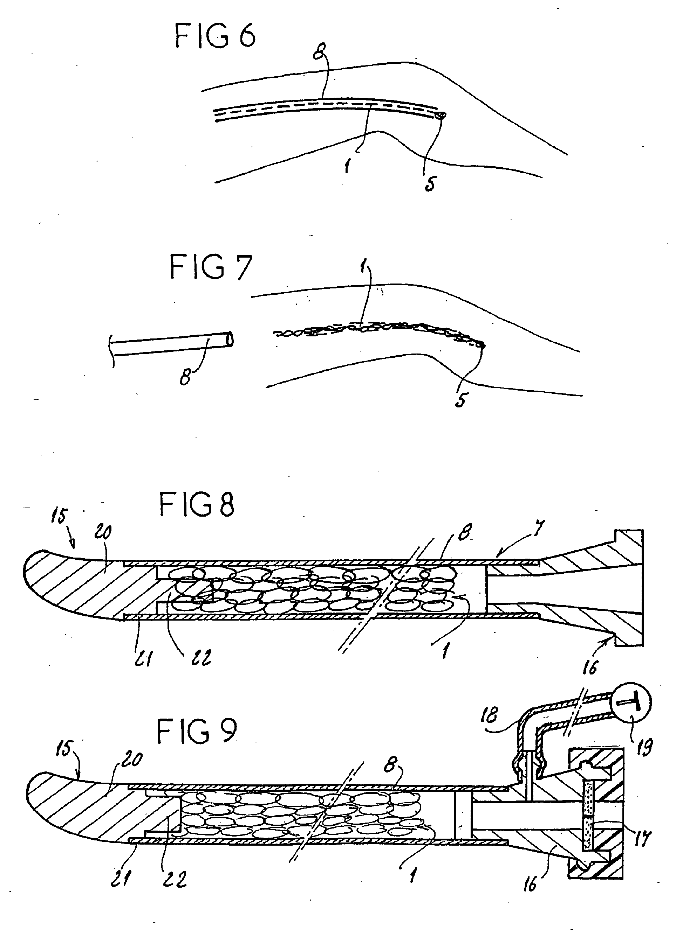

La figure 8 montre schématiquement un dispositif 1 identique à celui qui vient d'être décrit, sinon qu'il ne présente pas la zone 5, et un dispositif 7 comprenant, outre le tube 8, un embout distal 15 et un embout proximal 16.

L'embout 15 présente une portion arrondie 20, conformée pour être atraumatique, une portion circulaire 21 de diamètre légèrement inférieur au diamètre interne du tube 8, et une patte axiale 22 percée d'un trou. La portion 21 peut être engagée dans le tube 8 et permet le montage de l'embout 15 sur l'extrémité distale du tube 8, tandis que la patte 22 permet de lier cet embout 15 au dispositif 1.

Cet embout 15 remplit la même fonction que la zone 5 précitée. Après retrait du tube 8, il est extrait du corps du patient par une incision distale et une séparation d'avec le dispositif 1. Il peut également être réalisé en un matériau résorbable à résorption rapide et être alors laissé en place.

L'embout 16 est, dans l'exemple représenté sur la figure 8, de type "Luer lock". Il peut également être de type raccord en "Y" avec des extrémités au standard "Luer lock". Cet embout 16 permet une injection de fluide à l'intérieur du tube 8, soit préalablement à la mise en place du dispositif 1, soit pendant cette mise en place.

La figure 9 montre un dispositif 7 identique à celui de la figure 8 mais avec, à son extrémité proximale, un embout 16 de type embase avec valve hémostatique 17 et une voie latérale 18 terminée en son extrémité par un robinet 19 à deux ou trois voies. L'injection de fluide se fait par cette voie latérale.

Un produit propre à provoquer une sclérose de la veine 12 peut notamment être injecté de cette façon.

Ainsi qu'il apparaît de ce qui précède, l'invention apporte une amélioration déterminante à la technique antérieure, en fournissant un dispositif d'occlusion 1 d'un conduit corporel, notamment d'une veine variqueuse 12, qui est implantable selon une procédure relativement simple, rapide et peu onéreuse à mettre en oeuvre, cette procédure pouvant être mise en oeuvre sous anesthésie locale et n'induisant que peu de douleurs post-opératoires et pas de complications au niveau des tissus. Ce dispositif d'occlusion présente en outre des possibilités d'utilisation élargies, c'est-à-dire qu'il peut notamment être utilisé pour l'occlusion de la veine Saphène interne mais aussi de la veine Saphène externe et des veines collatérales de gros calibre.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, le dispositif d'occlusion peut comprendre six, huit ou dix fils notamment, le métier utilisé comprenant alors respectivement six, huit ou dix aiguilles et chaque fil sautant respectivement deux, trois ou quatre aiguilles au cours de chaque cycle ; le tricotage peut être à mailles jetées ou cueillies ; les aiguilles 3 peuvent être alimentées chacune par un fil 2 ou par plusieurs fils 2 si ces fils sont doubles ou triples ; les fils 2 peuvent recevoir un traitement de surface augmentant leur thrombogénéité ; le dispositif d'occlusion peut être réalisé en fils monofilaments ou en fils mono et multifilaments, de titres et de grosseurs variables selon le diamètre du dispositif d'occlusion à obtenir, ce diamètre étant adapté au calibre d'un type de veine à traiter.

## Revendications

1. Dispositif d'occlusion d'un conduit corporel, notamment d'une veine variqueuse, **caractérisé en ce qu'**il est formé par plusieurs fils (2) tricotés ensemble de manière à constituer un tricot allongé cylindrique, c'est-à-dire un tricot dans lequel les fils (2) formant les mailles se croisent sensiblement au niveau de la zone radialement interne de ce tricot, ledit tricot étant réalisé sur un métier circulaire à aiguilles, les fils sautant plusieurs aiguilles de telle sorte que les portions des fils situées entre ces aiguilles s'étendent au niveau de la zone radialement interne de la surface circulaire délimitée par les aiguilles, qui est également la zone radialement interne du dispositif.

2. Dispositif d'occlusion selon la revendication 1, **caractérisé en ce qu'**il est constitué de fils (2) en matériau résorbable, notamment en acide polylactique, polyglycolique ou un copolymère de ces derniers.

3. Dispositif d'occlusion selon la revendication 2, **caractérisé en ce que** le matériau résorbable constituant les fils (2), le nombre de ces fils (2) et le diamètre de ces fils (2) sont déterminés de telle sorte que la durée nécessaire à la résorption du dispositif d'occlusion soit supérieure ou égale à la durée de résorption naturelle du conduit corporel traité une fois l'occlusion réalisée.

4. Dispositif d'occlusion selon l'une des revendications 1 à 3, **caractérisé en ce qu'**il est réalisé en mailles jetées.

5. Dispositif d'occlusion selon l'une des revendications 1 à 4, **caractérisé en ce que** les fils (2) qui le forment sont enduits d'une ou plusieurs substances de nature à favoriser l'occlusion et/ou la dégénérescence du conduit corporel traité, ou reçoivent un traitement de surface augmentant leur thrombogénéité.

6. Dispositif d'occlusion selon l'une des revendications 1 à 5, **caractérisé en ce qu'**une thermofixation est réalisée pour limiter la déformabilité des mailles et éviter tout démaillage.

7. Dispositif d'occlusion selon l'une des revendications 1 à 6, **caractérisé en ce qu'**il comprend au moins un fil longitudinal relié à ses extrémités, formant un moyen anti-allongement du dispositif d'occlusion (1).

8. Dispositif de mise en place, dans le conduit corporel à traiter, du dispositif d'occlusion selon l'une des revendications 1 à 7, **caractérisé en ce qu'**il comprend ledit dispositif d'occlusion selon l'une des revendications 1 à 7 et un tube longitudinal (8) propre à contenir le dispositif d'occlusion (1), des moyens de préhension (5) étant prévus pour permettre la préhension du dispositif d'occlusion (1) au niveau d'une extrémité de ce tube (8), à savoir l'extrémité distale du tube (8) par rapport à l'orifice par lequel ce tube a été introduit dans le conduit corporel (12) à traiter.

9. Dispositif de mise en place selon la revendication 8, **caractérisé en ce que** le diamètre du tube (8) est inférieur à celui du dispositif d'occlusion (1) à l'état non comprimé de ce dernier.

10. Dispositif de mise en place selon la revendication 8 ou la revendication 9, **caractérisé en ce que** les moyens de préhension (5) sont constitués à partir des fils (2) formant le dispositif d'occlusion (1), notamment par nouage ou autre regroupement, en particulier fusion, de ces fils (2) au niveau de l'extrémité distale du dispositif d'occlusion (1).

11. Dispositif de mise en place selon la revendication 8 ou la revendication 9, **caractérisé en ce que** les moyens de préhension (5) sont constitués par un embout atraumatique (15) relié au dispositif d'occlusion (1), pouvant être monté à l'extrémité distale du tube (8).

12. Dispositif de mise en place selon l'une des revendications 8 à 11, **caractérisé en ce que** l'extrémité proximale du tube (8) comporte un embout de connexion (16), par exemple du type "Luer lock".

## Claims

1. A device for occlusion of a corporeal duct, in particular a varicose vein, **characterized in that** it is formed by several yarns (2) knitted together so as to constitute a cylindrical elongate knitted fabric, that is to say a knitted fabric in which the yarns (2) forming the stitches intersect substantially at the radially inner zone of this knitted fabric, said knitted fabric being done on a circular knitting machine with needles, the yarns jumping several needles so that the portions of yarns situated between these needles extend at the radially inner zone of the circular surface delimited by the needles, which is also the radially inner zone of the device.

2. The occlusion device as claimed in claim 1, **characterized in that** it is made up of yarns (2) of absorbable material, in particular of polylactic acid, polyglycolic acid, or a copolymer of these.

3. The occlusion device as claimed in claim 2, **characterized in that** the absorbable material constituting the yarns (2), the number of these yarns (2) and the diameter of these yarns (2) are determined in such a way that the period needed for absorption of the occlusion device is greater than or equal to the period of natural absorption of the treated corporeal duct once the occlusion has been performed.

4. The occlusion device as claimed in one of claims 1 through 3, **characterized in that** it is made of warp stitches.

5. The occlusion device as claimed in one of claims 1 through 4, **characterized in that** the yarns (2) forming it are coated with one or more substances of a nature to promote occlusion and/or degeneration of the treated corporeal duct, or their surface is treated to increase their thrombogenicity.

6. The occlusion device as claimed in one of claims 1 through 5, **characterized in that** thermosetting is performed in order to limit the deformability of the stitches and to prevent unraveling.

7. The occlusion device as claimed in one of claims 1 through 6, **characterized in that** it comprises at least one longitudinal yarn connected at its ends, forming a means acting against lengthening of the occlusion device (1).

8. A device for positioning the occlusion device as claimed in one of claims 1 through 7 in the corporeal duct to be treated, **characterized in that** it comprises said occlusion device according to one of claims 1 to 7 and a longitudinal tube (8) able to contain the occlusion device (1), grip means (5) being provided to permit gripping of the occlusion device (1) at one end of this tube (8), namely the distal end of the tube (8) in relation to the orifice through which this tube has been introduced into the corporeal duct (12) to be treated.

9. The positioning device as claimed in claim 8, **characterized in that** the diameter of the tube (8) is smaller than that of the occlusion device (1) in the uncompressed state of the latter.

10. The positioning device as claimed in claim 8 or claim 9, **characterized in that** the grip means (5) are constituted by the yarns (2) forming the occlusion device (1), in particular by knotting or other grouping together, in particular fusion, of these yarns (2) at the distal end of the occlusion device (1).

11. The positioning device as claimed in claim 8 or claim 9, **characterized in that** the grip means (5) are formed by an atraumatic endpiece (15) which is connected to the occlusion device (1), being able to be fitted at the distal end of the tube (8).

12. The positioning device as claimed in one of claims 8 through 11, **characterized in that** the proximal end of the tube (8) comprises a connector socket (16), for example of the Luer lock type.

## Patentansprüche

1. Vorrichtung zum Verschließen eines Körperkanals, insbesondere eines varikösen Blutgefäßes, **dadurch gekennzeichnet, dass** sie aus mehreren Fäden (2) gebildet ist, die insgesamt derart gewirkt sind, dass sie ein langerstrecktes zylindrisches Gewirk bilden, d.h. ein Gewirk, bei dem die Fäden (2), die die Maschen bilden, etwa im Bereich der radial inneren Zone des Gewirks verkreuzt sind, wobei das Gewirk auf einer Rundnadelwirkmaschine gefertigt ist, wobei die Fäden mehrere Nadeln derart überspringen, dass die sich zwischen den Nadeln befindenden Abschnitte der Fäden sich im Bereich der radial inneren Zone der von den Nadeln begrenzten kreisförmigen Oberfläche erstrecken, die auch die radial innere Zone der Vorrichtung ist.

2. Vorrichtung zum Verschließen nach Anspruch 1, **dadurch gekennzeichnet, dass** sie aus Fäden (2) aus einem resorbierbaren Material, insbesondere aus Polymilchsäure, Polyglycolsäure oder einem Copolymer von letzteren gebildet ist.

3. Vorrichtung zum Verschließen nach Anspruch 2, **dadurch gekennzeichnet, dass** das die Fäden (2) bildende resorbierbare Material, die Anzahl dieser Fäden (2) und der Durchmesser dieser Fäden (2) derart bestimmt sind, dass die für die Resorption der Vorrichtung zum Verschließen erforderliche Dauer größer als die oder gleich der Dauer der natürlichen Resorption des behandelten Körperkanals ist, sobald der Verschluss geschaffen ist.

4. Vorrichtung zum Verschließen nach einem der Ansprüche 1 bis 3, **dadurch gekennzeichnet, dass** sie aus Wurfmaschen gefertigt ist.

5. Vorrichtung zum Verschließen nach einem der Ansprüche 1 bis 4, **dadurch gekennzeichnet, dass** die sie bildenden Fäden (2) mit einer oder mehreren Substanzen einer Beschaffenheit beschichtet sind, die das Verschließen und/oder die Degeneration des behandelten Körperkanals begünstigt, oder eine Oberflächenbehandlung erhalten haben, die ihre Thrombogenität erhöht.

6. Vorrichtung zum Verschließen nach einem der Ansprüche 1 bis 5, **dadurch gekennzeichnet, dass** eine Wärmefixierung vorgenommen worden ist, um die Verformbarkeit der Maschen zu begrenzen und jegliche Entmaschung zu vermeiden.

7. Vorrichtung zum Verschließen nach einem der Ansprüche 1 bis 6, **dadurch gekennzeichnet, dass** sie zumindest einen an seinen Enden verbundenen längs verlaufenden Faden aufweist, der ein Mittel gegen eine Längung der Vorrichtung zum Verschließen (1) bildet.

8. Vorrichtung zum an Ort und Stelle Bringen der Vorrichtung zum Verschließen nach einem der Ansprüche 1 bis 7 in einem zu behandelnden Körperkanal, **dadurch gekennzeichnet, dass** sie die Vorrichtung zum Verschließen nach einem der Ansprüche 1 bis 7 und ein längs verlaufendes Rohr (8) aufweist, das geeignet ist, die Vorrichtung zum Verschließen (1) zu enthalten, wobei Greifmittel (5) vorgesehen sind, um das Greifen der Vorrichtung zum Verschließen (1) im Bereich eines Endes des Rohres (8) zu ermöglichen, nämlich des distalen Endes des Rohres (8) bezüglich der Öffnung, durch die das Rohr in den zu behandelnden Körperkanal (12) eingeführt worden ist.

9. Vorrichtung zum an Ort und Stelle Bringen nach Anspruch 8, **dadurch gekennzeichnet, dass** der Durchmesser des Rohrs (8) kleiner ist als derjenige der Vorrichtung zum Verschließen (1) im nichtkomprimierten Zustand der letzteren.

10. Vorrichtung zum an Ort und Stelle Bringen nach Anspruch 8 oder Anspruch 9, **dadurch gekennzeichnet, dass** die Greifmittel (5) ausgehend von den Fäden (2) gebildet sind, die die Vorrichtung zum Verschließen (1) bilden, insbesondere durch Verknoten oder eine andere Zusammenfassung, insbesondere Verschmelzung dieser Fäden (2) im Bereich des distalen Endes der Vorrichtung zum Verschließen (1).

11. Vorrichtung zum an Ort und Stelle Bringen nach Anspruch 8 oder Anspruch 9, **dadurch gekennzeichnet, dass** die Greifmittel (5) durch ein atraumatisches Ansatzstück (5) gebildet sind, das mit der Vorrichtung zum Verschließen (1) verbunden ist, und das am distalen Ende des Rohrs (8) angebracht sein kann.

12. Vorrichtung zum an Ort und Stelle Bringen nach einem der Ansprüche 8 bis 11, **dadurch gekennzeichnet, dass** das proximale Ende des Rohrs (8) ein Verbindungsansatzstück (16) aufweist, beispielsweise vom Typ "Luer-lock".
